(19) Europäisches Patentamt
European Patent Office
Office européen des brevets

(11) Publication number: 0 120 428 B1

(12) **EUROPEAN PATENT SPECIFICATION**

(45) Date of publication of patent specification: 23.05.90

(21) Application number: 84102963.0

(22) Date of filing: 17.03.84

(51) Int. Cl.[5]: C 07 D 405/04, A 61 K 31/445, A 61 K 31/40 // (C07D405/04, 311:68, 207:27),(C07D405/04, 311:68, 211:76)

(54) **Benzopyran isomers.**

(30) Priority: 24.03.83 GB 8308062

(43) Date of publication of application: 03.10.84 Bulletin 84/40

(45) Publication of the grant of the patent: 23.05.90 Bulletin 90/21

(84) Designated Contracting States: BE CH DE FR GB IT LI NL SE

(56) References cited:
EP-A- 76 075
EP-A- 99 12
EP-A-0 033 612
FR-A-2 468 601
GB-A-1 548 222

(73) Proprietor: BEECHAM GROUP PLC
Beecham House Great West Road
Brentford Middlesex TW8 9BD (GB)

(72) Inventor: Faruk, Erol
Merrilyn 26 Woodlands Road
Enfield Middlesex EN2 OLP (GB)

(74) Representative: Jones, Pauline et al
Beecham Pharmaceuticals Patents & Trade Marks Dept. Great Burgh Yew Tree Bottom Road
Epsom Surrey KT18 5XQ (GB)

EP 0 120 428 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to one isomeric form of a chemical compound having pharmacological activity, to a process for the preparation of such an isomer, to its pharmaceutical use, and to pharmaceutical compositions containing it.

EP—A—9912 discloses the compound, 6-cyano-3,4-dihydro-2,2-dimethyl-*trans*-4-pyrrolidino-2H-benzo[b]pyran-3-ol, which has blood pressure lowering activity. It also discloses that the (+)-isomer has greater blood pressure lowering activity than the (−)- isomer.

It has now been discovered that, in relation to a 3,4-dihydrobenzopyran-3-ol, characterised by being substituted in the 4-position by a pyrrolidon-1-yl radical *trans* to the 3-hydroxy group, one isomer, namely the (3S,4R)-isomer, has greater blood pressure lowering activity than the other isomer, namely the (3R,4S)-isomer.

Accordingly, the present invention provides the (3S,4R)-isomer of a compound of formula (I) as hereinafter defined, optionally in admixture with up to 45% of the mixture by weight of the corresponding (3R,4S)-isomer.

The (more active) (3S,4R)-isomer of the compound of formula (I) is the enantiomer of a compound of formula (I) which has a negative optical rotation:

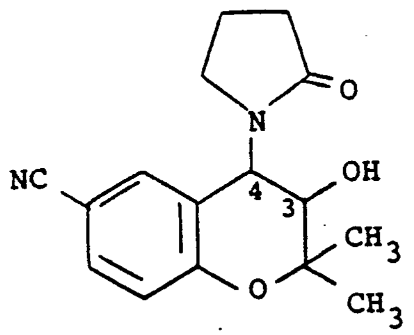

(I)

the OH and pyrrolidon-1-yl groups in formula (I) being mutually *trans*. The compound of formula (I) is trans-6-cyano-3,4-dihydro-2,2-dimethyl-4-(2-oxo-1-pyrrolidinyl)-2H-benzo[b]pyran-3-ol.

The other isomer of a compound of formula (I), i.e. the (3R,4S)-isomer, is the other enantiomer of the compound of formula (I), that is the enantiomer which has a positive optical rotation.

The absolute configuration of each isomer of a compound of formula (I) at the 3- and 4-centres may conveniently be determined by routine and conventional X-ray crystallographic analysis of an isolated diastereomeric derivative of that isomer, the configration at the 3- and 4- centres of the isomer and its derivative being the same. For example, an isomer of a compound of formula (I) may be reacted with a chiral esterifying agent with retention of 3- and 4- centre configuration to form a diastereomeric 3-ester derivative of the isomer. This may be isolated as a crystalline solid and the crystals used for the foregoing X-ray analysis.

A process for the preparation of diastereomeric derivatives of the isomers of a compound of formula (I), and for the resolution of the isomers of a compound of formula (I), is described hereinafter.

Preferably, the (3S,4R)-isomer of a compound of formula (I) is in a form containing from 0 to 40%, 0 to 30%, 0 to 20% or 0 to 10% of the corresponding (3R,4S)-isomer. Most preferably, the (3S,4R)-isomer is in a form containing 0% or no detectable amount of the corresponding (3R,4S)-isomer. All percentages hereinbefore are percentages of the mixture by weight. The presence of (3S,4R)-isomer may for example be routinely detected by the comparison of the optical rotation of a sample of the isomeric mixture with that of a pure sample of the (3S,4R)-isomer, or by the $^1H$ nmr spectrum of a sample of the isomeric mixture in the presence of a chiral shift reagent or chiral solvating agent.

The present invention also provides a process for the preparation of the (3S,4R)-isomer of a compound of formula (I), optionally in admixture with up to 45% of the mixture by weight of the corresponding (3R,4S)-isomer, which process comprises resolution of a mixture of (3S,4R)- and (4S,3R)-isomers of a compound of formula (I) to the extent that the resolution fraction which predominantly contains (3S,4R)-isomer optionally contains up to 45% by weight of that resolution fraction by weight of the corresponding (3R,4S)-isomer.

The term 'resolution' is used herein in the conventional practical sense used in the art to include partial resolution, that is, the separation of a mixture of enantiomers of a compound (in any ratio) into two fractions, one of which is enriched in one enantiomer relative to the initial mixture.

Resolution of the mixture of (3S,4R)- and (3R,4S)-isomers of compound of formula (I) may be effected conventionally by derivatising the mixture with a chiral derivatising agent, to form a mixture of diastereomers of a derivative of the compound of formula (I). The components of the mixture may then be separated conventionally, for example by fractional crystallisation. Separation may be complete, or partial, such that a given separated fraction comprises up to 45% of the total fraction by weight of the

diastereomeric derivative of the (3R,4S)-isomer. Subsequently, the diastereomeric derivative of the desired (3S,4R)-isomer, optionally in admixture with the diastereomeric derivative of the (3R,4S)-isomer is converted to the desired (3S,4S)-isomer of the compound of formula (I), optionally in admixture with up to 45% of the (3R,4S)-isomer.

The derivation for the process of the present invention is preferably effected on a mixture of (3S,4R)- and (3R,4S)-isomers of a compound of formula (I), by reacting such a mixture of isomers with an optically active reagent suitable for resolving alcohols. Such reagents include optically active acids or acid derivatives, such as acid chlorides, acid anhydrides and isocyanates. One preferred reagent is (−)-α-methylbenzyl isocyanate.

The resolution process is carried out in accordance with conventional procedures. With the preferred reagent, (−)-α-methylbenzyl isocyanate, formation of the derivatives is preferably carried out by heating the isocyanate with the mixture of isomers at reflux temperature in an inert solvent such as toluene. The resulting residue, which is a mixture of carbamates, may then separate conventionally for example by fractional crystallisation using a suitable solvent or solvent mixture, such as ethyl acetate and pentane.

In this way the diastereomeric derivative of the (3S,4R)-isomer may be obtained as a crystalline material, optionally in admixture with the diastereomeric derivative of the (3R,4S)-isomer, depending on the process conditions, and the remainder of the diastereomeric derivative of the (3R,4S)-isomer remains in the mother liquor. It is preferred that process conditions are chosen such that, if present, the diastereomeric derivative of the (3R,4S)-isomer is only 45% or less of the total crystalline fraction, so that the fraction may be converted to give the desired (3S,4R)-isomer of the compound of formula (I) in the desired form as hereinbefore defined without the need for further fractional crystallisation.

It will be appreciated that the optically active reagent could be such that the derivative of the (3R,4S)-isomer of a compound of formula (I) is initially isolated as crystalline material and the derivative of the desired (3S,4R)-isomer remains in the mother liquor, optionally with the derivative of the (3R,4S)-isomer also in the mother liquor. The derivative of the (3S,4R)-isomer optionally in admixture with the derivative of the (3R,4S)-isomer may be isolated from the mother liquor by conventional means such as further crystallisation. It is preferred that process conditions are chosen such that, if present, the derivative of the (3R,4S)-isomer is only present as 45% or less by weight of the total derivative of the compound of formula (I) present in the mother liquor, so that the crystals subsequently isolated from the mother liquor may be converted to give the desired (3S,4R)-isomer of the compound of formula (I) in the desired form as hereinbefore defined without the need for further fractional crystallisation.

It is preferred that separation of any diastereomeric derivatives is as complete as possible, so that the desired end-product (3S,4R)-isomer of a compound of formula (I) is in a form containing as little as possible of the (3R,4S)-isomer.

Conversion of the (partially) separated diastereomeric derivative of the desired (3S,4R)-isomer of the compound of formula (I) to the (3S,4R)-isomer of the compound of formula (I) in the desired form as hereinbefore defined may be effected conventionally. For example, when the diastereomeric derivatives are esters, these may be hydrolysed conventionally, for example by warming them with trichlorosilane in the presence of a base, such as triethylamine, in an inert solvent such as toluene.

The mixture of (3S,4R)- and (3R,4S)-isomers used in the resolution process of the present invention normally contains at least about 50% of (3R,4S)-isomer. In fact, mixtures containing about 50% of each isomer are usually obtained from any one of the processes described hereinafter for the preparation of a compound of formula (I).

The (3R,4S)-isomer corresponding to the desired end-product (3S,4R)-isomer of the compound of formula (I) may be incorporated into a mixture with the (3S,4R)-isomer to the extent that it forms up to 45% of the mixture by weight. The (3R,4S)-isomer may be incorporated into the mixture at any stage in the process of the invention subsequent to (partial) resolution step.

Thus the (3R,4S)-isomer corresponding to the desired (3S,4R)-isomer of the end-product compound of formula (I) may be added to that (3S,4R)-isomer (optionally already in admixture with (3R,4S)-isomer as hereinbefore defined), to the extent hereinbefore defined.

Alternatively, the (3R,4S)-isomer of an intermediate compound of formula (I) may be added to the corresponding (partially) resolved (3S,4R)-isomer, to the extent that it forms up to 45% of the mixture by weight.

It is preferred that there is no such subsequent incorporation of any such (3R,4S)-isomer corresponding to the desired (3S,4R)-isomer into a mixture therewith, so that the desired (3S,4R)-isomer of a compound of formula (I) is in a form containing as little as possible of the (3R,4S)-isomer.

A mixture of isomers, usually in an appropriate 50:50 ratio, of a compound of formula (I), may be obtained by carrying out any one of the processes 1 to 4 described in EP—A—120428.

The (3S,4R)-isomer of a compound of formula (I) has a better blood pressure lowering activity than the corresponding (3R,4S)-isomer. This isomer is therefore useful in the treatment of hypertension, optionally in admixture with the corresponding (3R,4S)-isomer as hereinbefore defined.

The present invention accordingly provides a pharmaceutical composition which comprises the (3S,4R)-isomer of a compound of formula (I) optionally in admixture with up to 45% of the mixture of isomers by weight of the corresponding (3R,4S)-isomer and a pharmaceutically acceptable carrier. In particular, the present invention provides an antihypertensive pharmaceutical composition which

comprises an antihypertensive effective amount of the (3S,4R)-isomer of a compound of formula (I) optionally in admixture with up to 45% of the mixture of isomers by weight of the corresponding (3R,4S)-isomer and a pharmaceutically acceptable carrier.

The compositions are preferably adapted for oral administration. However, they may be adapted for other modes of administration, for example parenteral administration for patients suffering from heart failure.

The compositions may be in the form of tablets, capsules, powders, granules, lozenges, suppositories, reconstitutable powders, or liquid preparations, such as oral or sterile parenteral solutions or suspensions.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The solid oral compositions may be prepared by conventional methods of blending, filling, tabletting or the like. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acaacia; nonaqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents. For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and buffering agents can be dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

The compositions may contain from 0.1% to 99% by weight, preferably from 10—60% by weight, of the active material, depending on the method of administration.

The isomers of the present invention can be used in the prophylaxis or treatment of hypertension in mammals including man, which comprises administering to the suffering mammal an anti-hypertensive effective amount of the (3S,4R)-isomer of a compound of formula (I) optionally in admixture with up to 45% of the mixture of isomers by weight of the corresponding (3R,4S)-isomer, or a pharmaceutical composition of the invention.

An effective amount will depend on the relative efficacy of the compounds of the present invention, the severity of the hypertension being treated and the weight of the sufferer. However, a unit dose form of a composition of the invention may contain from 0.5 to 100 mg of a compound of the invention and more usually from 1 to 50 mg, for example 1 to 10 mg, such as 1, 2, 5 or 10 mg. Such compositions may be administered from 1 to 6 times a day, more usually from 1 to 4 times a day, in a manner such that the daily dose is from 1 to 100 mg for a 70 kg human adult and more particularly from 1 to 10 mg.

The present invention further provides the (3S,4R)-isomer of a compound of formula (I) optionally in admixture with up to 45% of the mixture of isomers by weight of the corresponding (3R,4S)-isomer for use in the treatment of prophylaxis or hypertension.

The following example illustrates the resolution of the (3S,4R)-isomer of the compound of formula (I).

## Example

Resolution of 6-cyano-3,4-dihydro-2,2-dimethyl-trans-4-(2-oxo-1-pyrrolidinyl)-2H-benzo[b]pyran-3-ol (1)

A mixture of the title compound (16.6 mM) and (−)-α-methylbenzyl isocyanate (19.3 mM) in toluene (160 ml) was refluxed for 43 h and then evaporated to leave a gum. Fractional crystallisation from ethyl acetate-pentane afforded the carbamate of the (−)-isomer of the title compound, $[\alpha]_D^{26}$(acetone)−14.24°, in 21% yield. Crystallisation of the oil derived from the mother liquor similarly gave the carbamate of the (+) isomer of the title compound $[\alpha]_D^{26}$(acetone)−17.12°, in 14% yield.

The foregoing carbamates were each hydrolysed back to the parent carbinols by treatment with

triethylamine (two equivalents) and trichlorosilane (two equivalents) in toluene at 35—40°C for 16 h. The crude products were purified by chromatography on silica gel, eluting with ethyl acetate, to afford the pure (−)-isomer of the title compound $[\alpha]_D^{26}(CHCl_3)-52.2°$, m.p. 242—4°C (63%), and (+)-isomer of the title compound $[\alpha]_D^{26}(CHCl_3)$ +53.5°, m.p. 243—5°C (66%) respectively.

*Pharmacological Data*

Systolic blood pressures were recorded by a modification of the tail cuff method described by I. M. Claxton, M. G. Palfreyman, R. H. Poyser, R. L. Whiting, European Journal of Pharmacology, *37,* 179 (1976).

W+W BP recorder, model 8005, was used to display pulses. Prior to all measurements rats were placed in a heated environment (33.5 ± 5°C) before transfer to a restraining cage. Each determination of blood pressure was the mean of at least 6 readings. Spontaneously hypertensive rats (ages 12—18 weeks) with systolic blood pressures >170 mmHg were considered hypertensive.

| Compound (1) of Example (3S,4R)-isomer | Time Post Dose Hours | %Change in Systolic Blood Blood | %Change in Heart Rate |
|---|---|---|---|
| 5 rats | | | |
| Dose 0.3 mg/kg | 1 | -59 ± 4 | -2 ± 2 |
| p.o. | 2* | -52 ± 7 | -6 ± 3 |
| Initial Blood | | | |
| pressure | 4** | -32 | -15 |
| 180±7mmHg | 6* | -28 ± 8 | -5 ± 1 |
| Initial Heart | | | |
| Rate | 24*** | -14 ± 7 | -3 ± 2 |
| 507±5beats/min | | | |

* 2 rats had no measurable pulse

** 3 rats had no measurable pulse

*** 4 rats only, were used to determine heart rate, and blood pressure.

*Toxicity*

No toxic effects were observed in the above tests.

**Claims**

1. The (3S,4R)-isomer of a compound of formula (I) optionally in admixture with up to 45% of the mixture by weight of the corresponding (3R,4S)-isomer:

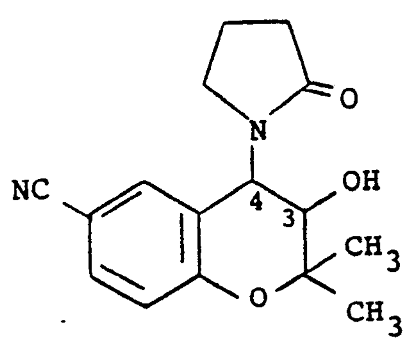

(I)

the OH and pyrrolidin-1-yl groups in formula (I) being mutually *trans*; and which is *trans*-6-cyano-3,4-dihydro-2,2-dimethyl-4-(2-oxo-1-pyrrolidinyl)-2H-benzo[b]pyran-3-ol.

2. The (3S,4R)-isomer of a compound of formula (I) as defined in claim 1, optionally in admixture with up to 20% of the mixture by weight of the corresponding (3R,4S)-isomer.

3. The (3S,4R)-isomer of a compound of formula (I) as defined in claim 1, optionally in admixture with up to 10% of the mixture by weight of the corresponding (3R,4S)-isomer.

4. The (3S,4R)-isomer of a compound of formula (I) as defined in claim 1, in the form defined in claims 1 to 3, wherein the (3S,4R)-isomer is in admixture with 0% or no detectable amount of the corresponding (3R,4S)-isomer.

5. A process for the preparation of the (3S,4R)-isomer of a compound of formula (I) according to claim 1, optionally in admixture with up to 45% of the mixture by weight of the corresponding (3R,4S)-isomer, which process comprises resolution of a mixture of (3S,4R)- and (4S,3R)-isomers of a compound of formula (I) as in claim 1 to the extent that the resolution fraction which predominantly contains (3S,4R)-isomer optionally contains up to 45% of that resolution fraction by weight of the corresponding (3R,4S)-isomer.

6. A process according to claim 5, wherein the mixture of (3S,4R)- and (3R,4S)-isomers of a compound of formula (I) is completely resolved, to give a (3S,4R)-isomer of a compound of formula (I), according to claim 1, in the form defined in claim 4.

7. A pharmaceutical composition which comprises the (3S,4R)-isomer of a compound of formula (I), according to claim 1, optionally in admixture with up to 45% of the mixture of isomers by weight of the corresponding (3R,4S)-isomer. and a pharmaceutically acceptable carrier.

8. A composition according to claim 7 wherein the (3S,4R)-isomer is in admixture with 0% or no detectable amount of the corresponding (3R,4S)-isomer.

9. The (3S,4R)-isomer of a compound of formula (I), according to claim 1, in the form defined in any one of claims 1 to 4, for use as an active therapeutic substance.

10. The (3S,4R)-isomer of a compound of formula (I), according to claim 1, in the form defined in any one of claims 1 to 4, for use in the treatment or prophylaxis of hypertension.

11. Use of an isomer according to any one of claims 1 to 4, in the manufacture of a medicament for use in the treatment or prophylaxis of hypertension.

12. The carbonate formed between (−)-6-cyano-3,4-dihydro-2,2-dimethyl-trans-4-(2-oxo-1-pyrrolidinyl)-2H-benzo[b]pyran-3-ol and (−)-α-methylbenzyl isocyanate.

**Patentansprüche**

1. Das (3S,4R)-Isomer einer Verbindung der Formel (I), gegebenenfalls im Zumischung mit bis zu 45 Gewichtsprozent der Mischung des entsprechenden (3R,4S)-Isomers:

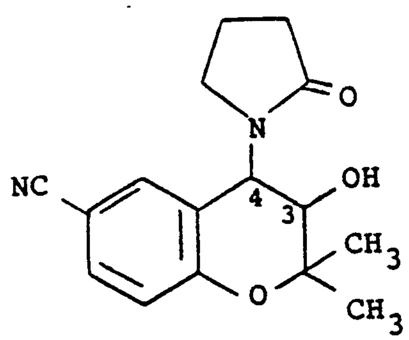

(I)

wobei die OH— und Pyrrolidon-1-ylgruppen in Formel (I) sich in wechselseitiger trans-Stellung befinden; und welches trans-6-Cyano-3,4-dihydro-2,2-dimethyl-4-(2-oxo-1-pyrrolidinyl)-2H-benzo[b]pyran-3-ol ist.

2. Das (3S,4R)-Isomer einer Verbindung der Formel (I), wie in Anspruch 1 definiert, gegebenenfalls in Zumischung mit bis zu 20 Gewichtsprozent der Mischung des entsprechenden (3R,4S)-Isomers.

3. Das (3S,4R)-Isomer einer Verbindung der Formel (I), wie in Anspruch 1 definiert, gegebenenfalls in Zumischung mit bis zu 10 Gewichtsprozent der Mischung des entsprechenden (3R,4S)-Isomers.

4. Das (3S,4R)-Isomer einer Verbindung der Formel (I), wie in Anspruch 1 definiert, in der Form, wie in den Ansprüchen 1 bis 3 definiert, in welchem das (3S,4R)-Isomer in Zumischung mit 0% bzw. keiner erfaßbaren Menge des entsprechenden (3R,4S)-Isomers vorliegt.

5. Ein verfahren zur Herstellung des (3S,4R)-Isomers einer Verbindung der Formel (I) nach Anspruch 1, gegebenenfalls in Zumischung mit bis zu 45 Gewichtsprozent der Mischung des entsprechenden (3S,4R)-Isomers, welches Verfahren das Auftrennen einer Mischung von (3S,4R)- und (4S,3R)-Isomer einer Verbindung der Formel (I), wie in Anspruch 1, in dem Maße umfaßt, daß die durch Auftrennen erhaltene Fraktion, welche hauptsächlich (3S,4R)-Isomer enthält, gegebenenfalls bis zu 45 Gewichtsprozen dieser Fraktion des entsprechenden (3R,4S)-Isomers enthält.

6. Ein Verfahren nach Anspruch 5, in welchem die Mischung der (3S,4R)- und (3R,4S)-Isomer einer Verbindung der Formel (I) vollständig aufgetrennt wird unter Erhalt eines (3S,4R)-Isomers einer Verbindung der Formel (I), nach Anspruch 1, in der in Anspruch 4 definierten Form.

7. Eine pharmazeutische Zusammensetzung, welche das (3S,4R)-Isomer einer Verbindung der Formel (I), nach Anspruch 1, gegebenenfalls in Zumischung mit bis zu 45 Gewichtsprozen der Isomerenmischung des entsprechenden (3R,4S)-Isomers, und einen pharmazeutisch verträglichen Träger umfaßt.

8. Eine Zusammensetzung nach Anspruch 7, in welcher das (3S,4R)-Isomer in Zumischung mit 0% bzw. einer nicht feststellbaren Menge des entsprechenden (3R,4S)-Isomers vorliegt.

9. Das (3S,4R)-Isomer einer Verbindung der Formel (I), nach Anspruch 1, in der in einem der Ansprüche 1 bis 4 definierten Form, zu Verwendung als aktive therapeutische Substanz.

10. Das (3S,4R)-Isomer einer Verbindung der Formel (I), nach Anspruch 1, in der in einem der Ansprüche 1 bis 4 definierten Form, zur Verwendung bei der Behandlung oder Prophylaxe von Bluthochdruck.

11. Verwendung eines Isomers nach einem der Ansprüche 1 bis 4, bei der Herstellung eines Medikaments zur Verwendung bei der Behandlung oder Prophylaxe von Bluthochdruck.

12. Das zwischen (−)-6-Cyano-3,4-dihydro-2,2-dimethyl-trans-4-(2-oxo-1-pyrrolidinyl)-2H-benzo[b]pyran-3-ol und (−)-α-Methylbenzylisocyanat gebildete Carbamat.

**Revendications**

1. Isomère (3S,4R) d'un composé de formule (I) caractéisé en ce qu'il est éventuellement en mélange avec jusqu'à 45% du mélange en poids de l'isomère (3R,4S) correspondant,

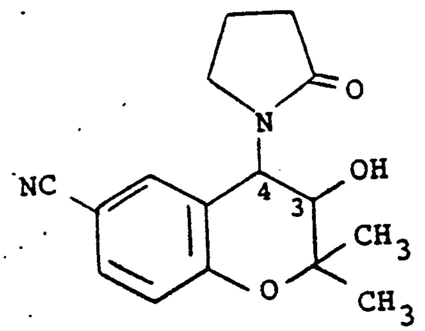

(I)

les groupes OH et pyrrolidon-1-yl dans la formule (I), étant mutuellement trans; et qu'il est le trans-6-cyano-3,4-dihydro-2,2-dyméthyl-4-(2-oxo-1-pyrrolidinyl-2H-benzo(b)pyran-3-ol.

2. Isomère (3S,4R) d'un composé de formule (I) suivant la revendication 1, caractérisé en ce qu'il est éventuellement en mélange avec jusqu'à 20% de mélange en poids de l'isomère (3R,4S) correspondante.

3. Isomère (3S,4R) d'un composé de formule (I) suivant la revendication 1, caractérisé en ce qu'il est éventuellement en mélange avec jusqu'à 10% de mélange en poids de l'isomère (3R,4S) correspondante.

4. Isomère (3S,4R) d'un composé de formule (I) suivant la revendication 1, sous la forme définie dans les revendications 1 à 3, caractérisé en ce que l'isomère (3S,4R) est en mélange avec une quantité de 0% ou non détectable de l'isomère (3R,4S) correspondant.

5. Procédé de préparation de l'isomère (3S,4R) d'un composé de formule (I) suivant la revendication 1, éventuellement en mélange avec une quantité allant jusqu'à 45% du mélange en poids de l'isomère (3R,4S) correspondant, caractérisé en ce qu'il comprend la résolution d'un mélange des isomères (3S,4R) en (4S,3R) d'un composé de formule (I) suivant la rvendication 1 jusqu'au point où la fraction de résolution qui contient principalement l'isomère (3S,4R) renferme éventuellement jusqu'à 45% de cette fraction de résolution en poids de l'isomère (3R,4S) correspondant.

6. Procédé suivant la revendication 5, caractérisé en ce que le mélange d'isomères (3S,4R) et (3R,4S) d'un composé de formule (I) est complétement résolu pour donner un isomère (3S,4R) d'un composé de formule (I) suivant la revendication 1, sous la forme définie dans la revendication 4.

7. Composition pharmaceutique caractérisée en ce qu'elle comprend l'isomère (3S,4R) d'un composé de formule (I) suivant la revendication 1, éventuellement en mélange avec jusqu'à 45% du mélange d'isoméres en poids de l'isomère (3R,4S) correspondant, est un support acceptable du point de vue pharmaceutique.

8. Composition suivant la revendication 7, caractérisé en ce que l'isomère (3S,4R) est en mélange avec une quantité de 0% ou non détectable de l'isomère (3R,4S) correspondant.

9. Isomère (3S,4R) d'un composé de formule (I) suivant la revendication 1 sous la forme définie suivant l'une quelconque des revendications 1 à 4, caractérisé en ce qu'il est utilisé en tant que substance thérapeutique active.

10. Isomère (3S,4R) d'un composé de formule (I) suivant la revendication 1, sous la forme définie dans

l'une quelconque des revendications 1 à 4, caractérisé en ce qu'il est utilisé dans le traitement ou la prophylaxie de l'hypertension.

11. Utilisation d'un isomère suivant l'une quelconque des revendications 1 à 4, dans la fabrication d'un médicament utile dans le traitement ou la prophylaxie de l'hypertension.

12. Carbamate, caractérisé en ce qu'il est formé à partir du (−)-6-cyano-3,4-dihydro-2,2-diméthyl-trans-4-(2-oxo-1-pyrrolidinyl)-2H-benzo(b)pyran-3-ol et de l'isocyanate d (−)-a-méthyl benzyle.